(19) Europäisches Patentamt
European Patent Office
Office européen des brevets

(11) Veröffentlichungsnummer: **0 302 327**
**A1**

(12) # EUROPÄISCHE PATENTANMELDUNG

(21) Anmeldenummer: **88111845.9**

(22) Anmeldetag: **22.07.88**

(51) Int. Cl.4: **C07D 231/18 , A01N 43/56 , //C07C149/14,C07C109/04, C07C25/13**

(30) Priorität: **03.08.87 DE 3725660**

(43) Veröffentlichungstag der Anmeldung: **08.02.89 Patentblatt 89/06**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Wroblowsky, Heinz-Jürgen, Dr.**
**Gladbacher Strasse 34**
**D-4018 Langenfeld(DE)**
Erfinder: **Marhold, Albrecht, Dr.**
**Carl-Duisberg-Strasse 329**
**D-5090 Leverkusen 1(DE)**
Erfinder: **Becker, Benedikt, Dr.**
**Metzkausener Strasse 14**
**D-4020 Mettmann(DE)**
Erfinder: **Behrenz, Wolfgang, Dr.**
**Untergründemich 14**
**D-5063 Overath(DE)**
Erfinder: **Homeyer, Bernhard, Dr.**
**Obere Strasse 28**
**D-5090 Leverkusen 3(DE)**
Erfinder: **Stendel, Wilhelm, Dr.**
**In den Birken 55**
**D-5600 Wuppertal 1(DE)**
Erfinder: **Andrews, Peter, Dr.**
**Gellertweg 2**
**D-5600 Wuppertal 1(DE)**

(54) **3,5-Dialkyl-I-arylpyrazole.**

(57) Es werden neue 3,5-Dialkyl-1-aryl-pyrazole der Formel (I)

EP 0 302 327 A1

Xerox Copy Centre

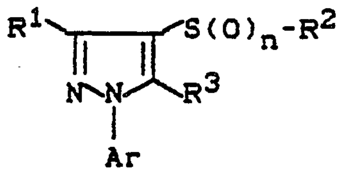

(I)

bereitgestellt,
in welcher
$R^1$ für Alkyl steht,
$R^2$ für Alkyl oder Halogenalkyl steht,
$R^3$ für Alkyl steht,
Ar für einen der Reste

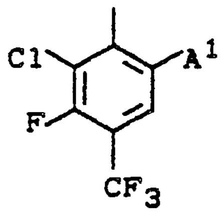 ; 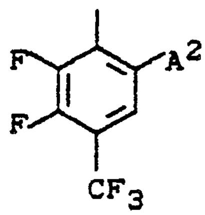 ; 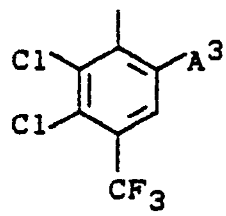 ;

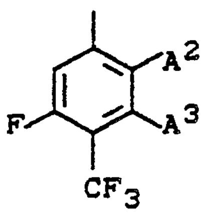 ; 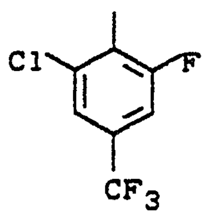 oder 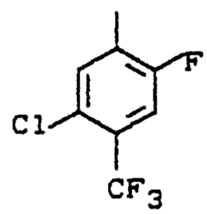

steht, wobei
$A^1$ für Wasserstoff, Fluor oder Chlor steht,
$A^2$ für Fluor oder Chlor steht,
$A^3$ für Wasserstoff oder Fluor steht und
n für eine Zahl 0, 1 oder 2 steht.

Die Verbindungen der Formel (I) besitzen eine ausgezeichnete Wirksamkeit gegen tierische Schädlinge, insbesondere gegen Insekten.

## 3,5-Dialkyl-1-arylpyrazole

Die Erfindung betrifft neue 3,5-Dialkyl-1-arylpyrazole, mehrere Verfahren zu ihrer Herstellung und ihre Verwendung als Schädlingsbekämpfungsmittel.

Es ist bereits bekannt, daß bestimmte 1-Arylpyrazole wie beispielsweise das 5-Methylamino-4-trifluormethylthio-1-(2,6-dichlor-4-trifluormethylphenyl)-pyrazol oder das 5-Methylamino-4-dichlorfluormethylthio-1-(2,6-dichlor-4-trifluormethylphenyl)-pyrazol insektizide Eigenschaften besitzen (vgl. EP 201 852).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue 3,5-Dialkyl-1-arylpyrazole der allgemeinen Formel (I),

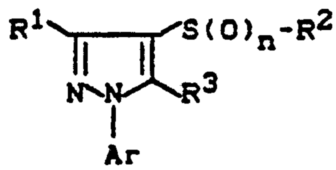

(I)

in welcher

$R^1$ für Alkyl steht,

$R^2$ für Alkyl oder Halogenalkyl steht,

$R^3$ für Alkyl steht,

Ar für einen der Reste

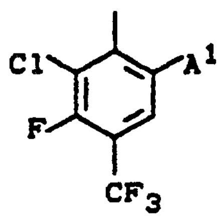 ; 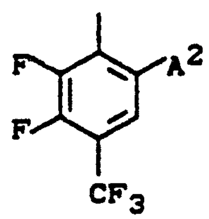 ; 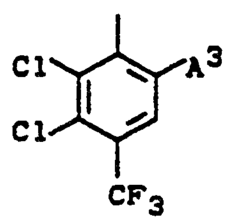 ;

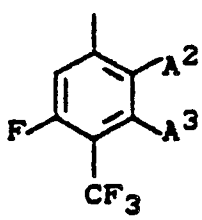 ; 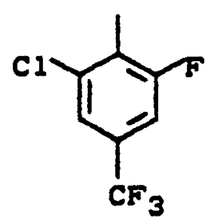 oder 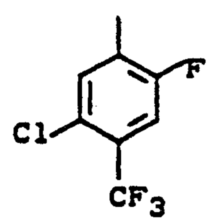

steht, wobei

$A^1$ für Wasserstoff, Fluor oder Chlor steht,

$A^2$ für Fluor oder Chlor steht,

$A^3$ für Wasserstoff oder Fluor steht und

n für eine Zahl 0, 1 oder 2 steht,

gefunden.

Weiterhin wurde gefunden, daß man die neuen 3,5-Dialkyl-1-arylpyrazole der allgemeinen Formel (I),

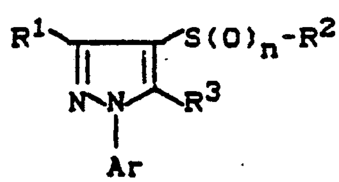

(I)

in welcher
$R^1$ für Alkyl steht,
$R^2$ für Alkyl oder Halogenalkyl steht,
$R^3$ für Alkyl steht,
Ar für einen der Reste

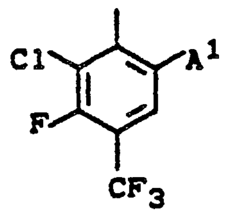 ; 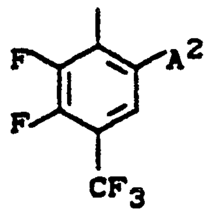 ; 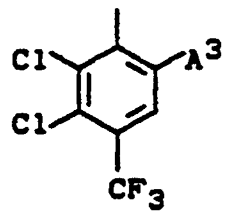 ;

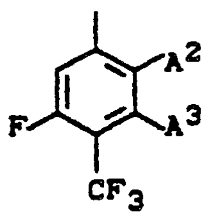 ; 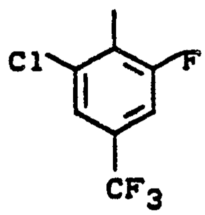 oder 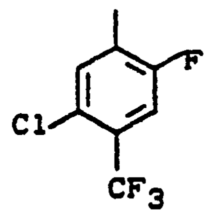

steht, wobei
$A^1$ für Wasserstoff, Fluor oder Chlor steht,
$A^2$ für Fluor oder Chlor steht,
$A^3$ für Wasserstoff oder Fluor steht und
n für eine Zahl 0, 1 oder 2 steht,
nach einem der im Folgenden beschriebenen Verfahren erhält:
Man erhält 3,5-Dialkyl-1-aryl-pyrazole der Formel (Ia),

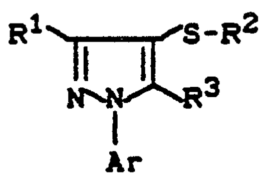 (Ia)

in welcher
$R^1$, $R^2$, $R^3$ und Ar die oben angegebene Bedeutung haben,
wenn man
a) Arylhydrazine der Formel (II),
$Ar\text{-}NH\text{-}NH_2$ (II)
in welcher
Ar die oben angegebene Bedeutung hat,
oder deren Säureadditionssalze mit 1,3-Diketonen der Formel (III),

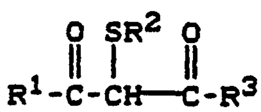 (III)

in welcher
$R^1$, $R^2$ und $R^3$ die oben angegebene Bedeutung haben,
gegebenenfalls in Gegenwart eines Verdünnungsmittels und gegebenenfalls in Gegenwart eines Katalysators umsetzt.

Man erhält 4-Sulfinyl- und 4-Sulfonyl-3,5-dialkyl-1-aryl-pyrazole der Formel (Ib),

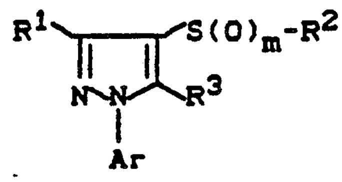

(Ib)

in welcher
$R^1$, $R^2$, $R^3$ und Ar die oben angegebene Bedeutung haben und
m für eine Zahl 1 oder 2 steht,
wenn man
b) die nach Verfahren (a) erhältlichen 3,5-Dialkyl-1-arylpyrazole 1-Arylpyrazole der Formel (Ia),

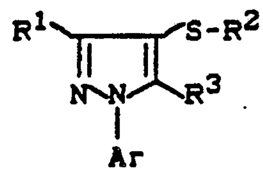

(Ia)

in welcher
$R^1$, $R^2$, $R^3$ und Ar die oben angegebene Bedeutung haben,
mit Oxidationsmitteln gegebenenfalls in Gegenwart eines Verdünnungsmittels und gegebenenfalls in Gegenwart eines Katalysators in üblicher Art und Weise oxidiert.

Schließlich wurde gefunden, daß die neuen 3,5-Dialkyl-1-arylpyrazole der allgemeinen Formel (I) pestizide und insbesondere insektizide Eigenschaften besitzen.

Überraschenderweise zeigen die erfindungsgemäßen 3,5-Dialkyl-1-arylpyrazole der allgemeinen Formel (I) eine erheblich bessere insektizide Wirksamkeit als die aus dem Stand der Technik bekannten 1-Arylpyrazole, wie beispielsweise das 5-Methylamino-4-trifluormethylthio-1-(2,6-dichlor-4-trifluormethyl-phenyl)-pyrazol oder das 5-Methylamino-4-dichlorfluormethylthio-1-(2,6-dichlor-4-trifluormethyl-phenyl)-pyrazol, welche chemisch und wirkungsmäßig naheliegende Verbindungen sind.

Die erfindungsgemäßen 3,5-Dialkyl-1-arylpyrazole sind durch die Formel (I) allgemein definert. Bevorzugt sind Verbindungen der Formel (I), bei welchen
$R^1$ für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für geradkettiges oder verzweigtes Alkyl mit 1 bis 6 Kohlenstoffatomen steht,
Ar für einen der Reste

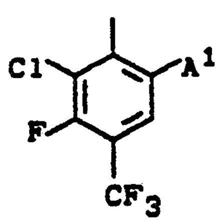 ; 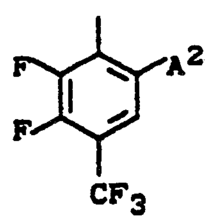 ; 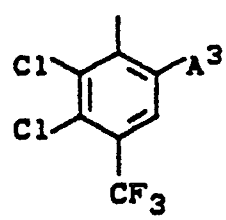 ;

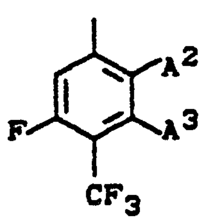 ; 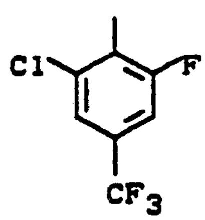 oder 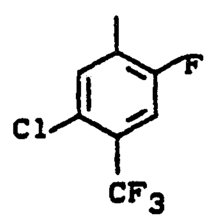

steht, wobei

$A^1$ für Wasserstoff, Fluor oder Chlor steht,

$A^2$ für Fluor oder Chlor steht,

$A^3$ für Wasserstoff oder Fluor steht und

n für eine Zahl 0, 1 oder 2 steht.

Besonders bevorzugt sind Verbindungen der Formel (I), bei welchen

$R^1$ für Methyl oder Ethyl steht,

$R^2$ für Methyl, Ethyl, n- oder i-Propyl, n-, i-, s-oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder Brompropyl steht,

$R^3$ für geradkettiges oder verzweigtes Alkyl mit 1 bis 4 Kohlenstoffatomen steht,

Ar für einen der Reste

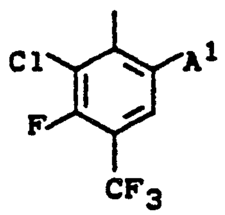 ; 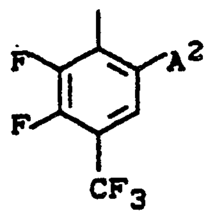 ; 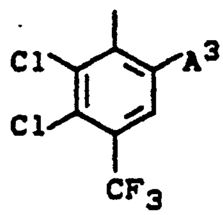 ;

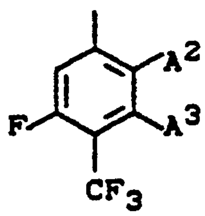 ; 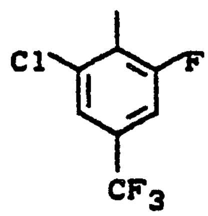 oder 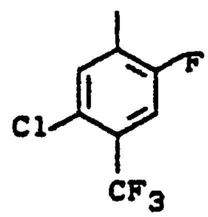

steht, wobei

$A^1$ für Wasserstoff, Fluor oder Chlor steht,

$A^2$ für Fluor oder Chlor steht,

$A^3$ für Wasserstoff oder Fluor steht und

n für eine Zahl 0, 1 oder 2 steht.

Ganz besonders bevorzugt sind Verbindungen der Formel (I), bei welchen

$R^1$ für Methyl steht,

$R^2$ für Methyl, Ethyl, Trifluormethyl, Dichlorfluormethyl oder Difluorchlormethyl steht,

$R^3$ für Methyl, Ethyl, n- oder i-Propyl steht,

Ar für einen der Reste

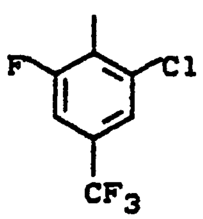 ; 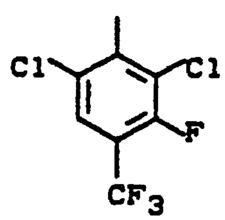 oder 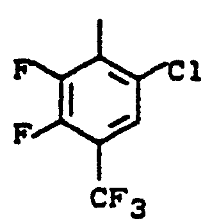 steht

und

n für eine Zahl 0, 1 oder 2 steht.

Verwendet man beispielsweise 2,6-Dichlor-3-fluor-4-trifluormethyl-phenylhydrazin und 3-Trifluormethylthiopentan-2,4-dion als Ausgangsstoffe, so läßt sich der Reaktionsablauf des erfindungsgemäßen Verfahrens (a) durch das folgende Formelschema darstellen:

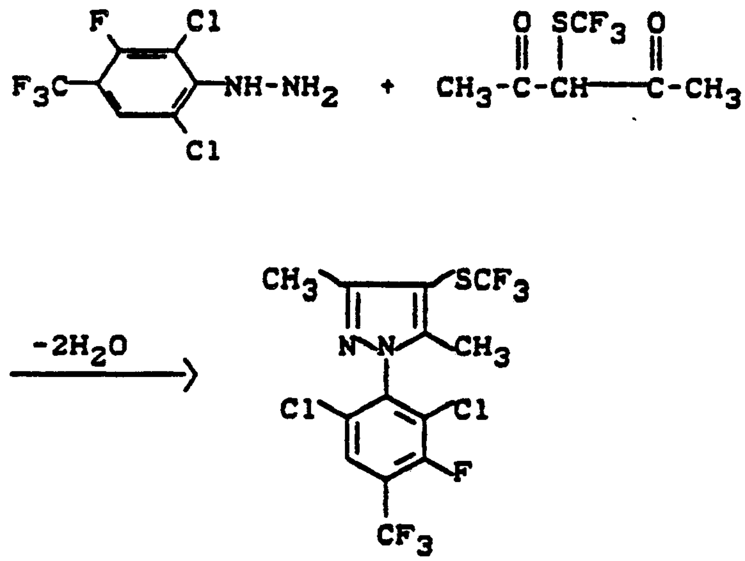


Verwendet man beispielsweise 1-(2-Chlor-6-fluor-4-trifluormethyl-phenyl)-3,5-dimethyl-4-trifluormethylthiopyrazol als Ausgangsverbindung und m-Chlorperbenzoesäure als Oxidationsmittel, so läßt sich der Reaktionsablauf des erfindungsgemäßen Verfahrens (b) durch das folgende Formelschema darstellen:

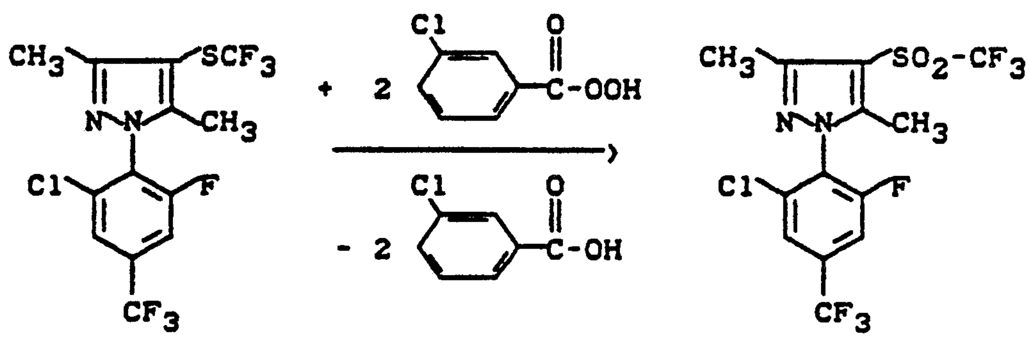


Die zur Durchführung des erfindungsgemäßen Verfahrens (a) als Ausgangsstoffe benötigten Arylhydrazine sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Arylhydrazine der Formel (II) sind noch nicht bekannt. Sie sind jedoch Gegenstand einer eigenen vorgängigen noch nicht publizierten Patentanmeldung (vgl. Deutsche Patentanmeldung P 3 617 977 vom 28.05.1986). Man erhält sie in Analogie zu bekannten Verfahren, beispielsweise wenn man Arylhalogenide der Formel (IV),

$Ar-Hal^1$ (IV)

in welcher

$Hal^1$ für Halogen, insbesondere für Chlor oder Fluor steht und

Ar die oben angegebene Bedeutung hat,

mit Hydrazinhydrat gegebenenfalls in Gegenwart eines Verdünnungsmittels wie beispielsweise Pyridin oder Dioxan bei Temperaturen zwischen 0 °C und +150 °C umsetzt.

Arylhalogenide der Formel (IV) sind zum Teil bekannt oder erhältlich in Analogie zu bekannten Verfahren (vgl. z.B. EP 187 023, EP 180 057, US 4 388 472; Zh. org. Khim. 20, 2187-2191 [1984] bzw. CA 102: 112 944s; J. Fluorine Chem. 4, 317-326 [1974]; J. chem. Soc. C, 1969, 211-217.

Weiterhin existiert ein nicht-naheliegendes Verfahren zur Herstellung von mit Fluor und gegebenenfalls zusätzlich Chlor substituierten Benzotrifluoriden der Formel (IV) bei welchem Fluor und Chlor enthaltende Benzotrifluoride in Gegenwart eines Katalysators und eines Chlorwasserstoffakzeptors hydriert werden.

Als Katalysatoren kommen im Prinzip bekannte Hydrierkatalysatoren in Frage, beispielsweise solche, welche die Elemente der VIII. Nebengruppe des Periodensystems in metallischer Form oder in Form von

Verbindungen enthalten. Insbesondere geeignet sind Nickel, Platin, Palladium und deren Verbindungen, beispielsweise in Form von Raney-Nickel, metallischem Platin, metallischem Palladium und Palladium-tetra-(triphenylphoshin). Die katalytisch aktive Substanz kann auch auf Trägermaterialien aufgebracht sein, beispielsweise auf Aktivkohle, Siliziumdioxid, Alumiumoxid, Silikaten oder Erdalkalisulfaten.

Bevorzugte Katalysatoren sind Raney-Nickel und metallisches Palladium auf Aktivkohle.

Die Katalysatoren können auch aus mehreren Komponenten zusammengesetzt sein und z.B. auch Promotoren enthalten, bei denen es sich auch um andere Elemente und Verbindungen als diejenigen der Metalle der VIII. Nebengruppe des Periodensystems handeln kann.

Die Menge des Katalysators ist im allgemeinen nicht kritisch. Sie kann beispielsweise 0,01 bis 15 Gew.-%, bezogen auf aktiven Katalysator betragen. Vorzugsweise beträgt diese Menge 0,1 bis 10 Gew.-% bezogen auf aktiven Katalysator (d.h. z.B. auf Pd, das auf A-Kohle aufgetragen ist.)

Die Hydrierung wird im allgemeinen in Gegenwart von Lösungs- odr Verdünnungsmitteln durchgeführt. Bei diesen ist es nicht zwingend erforderlich, daß sie die Einsatzmaterialien vollständig aufzulösen vermögen, da auch ein zweiphasiges Substrat hydriert werden kann. Beispielsweise können das Ausgangs-Benzotrifluorid und/oder der Chlorwasserstoff-Akzeptor während der Hydrierung ganz oder teilweise in suspendierter Form vorliegen. Geeignete Lösungs- und Verdünnungsmittel sind beispielsweise organische Säuren, wie Essigsäure; Alkohole, wie Methanol, Ethanol, Propanol und Isopropanol; Ether, wie Tetrahydrofuran; Nitrile, wie Acetonitril und Wasser.

Als Chlorwasserstoff-Akzeptoren kommen die verschiedensten anorganischen und organischen Basen in Frage, beispielsweise die Hydroxide, Carbonate, Acetate und Ammoniumsalze der Alkali- und Erdalkalimetalle und Amine, insbesondere tertiäre Amine. Bevorzugt sind Natriumacetat und Triethylamin, N-N-Dimethylanilin, Pyrimidin und Picolin.

Der Chlorwasserstoff-Akzeptor kann in verschiedenen Mengen eingesetzt werden. Vorzugsweise werden pro Äquivalent aus dem eingesetzten Benzotrifluorid abzuspaltender Chloratome mindestens 0,8 Äquivalente Chlorwasserstoff-Akzeptor eingesetzt. Wenn aus dem eingesetzten Benzotrifluorid alle vorhandenen Chloratome abgespalten werden sollen ist die Menge des einzusetzenden Chlorwasserstoff-Akzeptors nach oben hin nicht kritisch. Aus praktischen Erwägungen ist es im allgemeinen vorteilhaft, in diesem Fall nicht mehr als 2 Äquivalente Chlorwasserstoff-Akzeptor pro Äquivalent abzuspaltender Chloratome eizusetzen. Wenn aus dem eingesetzten Benzotrifluorid nicht alle vorhandenen Chloratome abgespalten, d.h. noch Chloratome enthaltende Benzotrifluoride hergestellt werden sollen, so darf nicht wesentlich mehr Chlorwasserstoff-Akzeptor eingesetzt werden als stöchiometrisch erforderlich ist. Vorzugs weise setzt man in diesem Fall bis zu 1,2, besonders bevorzugt bis 1,05 und ganz besonders bevorzugt 1 Äquivalent Chlorwasserstoff-Akzeptor pro Äquivalent abzuspaltende Chloratome ein.

Die erfindungsgemäße Hydrierung kann beispielsweise bei Drucken im Bereich von Normaldruck bis 200 bar und Temperaturen im Bereich von 20 bis 200 °C durchgeführt werden. Bevorzugt sind Drucke im Bereich Normaldruck bis 120 bar und Temperaturen im Bereich von 50 bis 140 °C.

Siehe dazu auch die Herstellungsbeispiele Nr. IV-3 bis IV-7 der vorliegenden Patentanmeldung. So können beispielsweise die folgenden neuen unter die Formel IV fallenden Ausgangsprodukte (A) und (B) hergestellt werden:

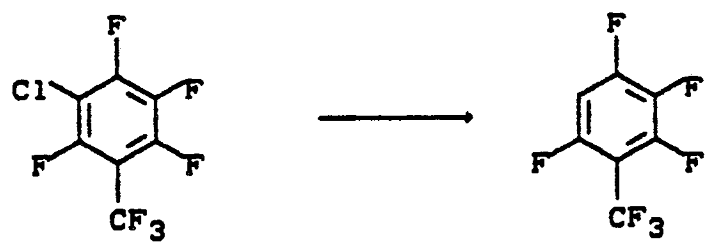

(A)

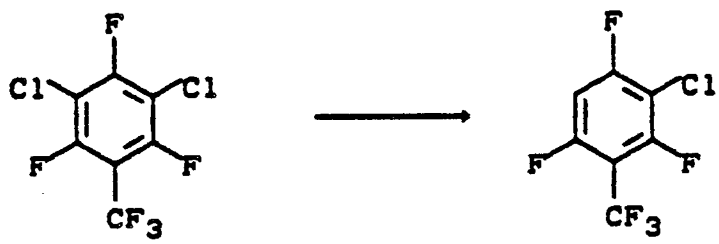

(B)

Die zur Durchführung des erfindungsgemäßen Verfahrens (a) weiterhin als Ausgansstoffe benötigten 1,3-Diketone sind durch die Formel (III) allgemein definiert. In dieser Formel (III) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1,3-Diketone der Formel (III) sind bekannt (vgl. z.B. DE-OS 2 155 493; DE-OS 2 455 568; J. Chromatography 230, 335-344 [1982]; Chem. Ber. 106, 1418-1422 [1973]; J. org. Chem. 38, 2809-2813 [1973]; J. org. Chem. 46, 153-157 [1981]; J. org. Chem. 49, 3494-3498 [1984]; DE-OS 2 138 728) oder lassen sich in Analogie zu bekannten Verfahren herstellen, beispielsweise, wenn man 1,3-Diketone der Formel (V),

$R^1$- C -$CH_2$- C -$R^3$ (V)
‖ ‖
O O

in welcher
$R^1$ und $R^3$ die oben angegebene Bedeutung haben,
mit Sulfenylchloriden der Formel (VIa),

$R^2$-S-Cl (VIa)

in welcher
$R^2$ die oben angegebene Bedeutung hat,
oder mit Alkansulfonsäurethioestern der Formel (VIb),

$R^4$-$SO_2$-S-$R^2$ (VIb)

in welcher
$R^2$ die oben angegebene Bedeutung hat und
$R^4$ für Alkyl, insbesondere für Methyl oder Ethyl steht,
gegebenenfalls in Gegenwart eines Verdünnungsmittels wie beispielsweise Toluol, Dioxan oder Methanol und gegebenenfalls in Gegenwart eines Säurebindemittels wie beispielsweise Natriummethylat oder Triethylamin bei Temperaturen zwischen 0 °C und 50 °C umsetzt;
oder wenn man 2-Halogen-1,3-diketone der Formel (VII),

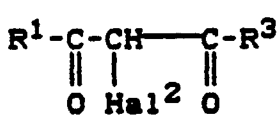

(VII)

in welcher
$R^1$ und $R^3$ die oben angegebene Bedeutung haben und
$Hal^2$ für Halogen, insbesondere für Chlor oder Brom steht,
mit Thiolen der Formel (VIII),

$R^2$-SH (VIII)

in welcher
$R^2$ die oben angegebene Bedeutung hat,
gegebenenfalls in Gegenwart eines Verdünnungsmittels wie beispielsweise Methanol oder Dimethylformamid und gegebenenfalls in Gegenwart eines Säurebindemittels wie beispielsweise Natriumhydroxid oder Natriummethylat bei Temperaturen zwischen 0 °C und 80 °C umsetzt.

Die 1,3-Diketone der Formel (V), die Sulfenylchloride der Formel (VIa), die Alkansulfonsäurethioester der Formel (VIb), die 2-Halogen-1,3-diketone der Formel (VII) und die Thiole der Formel (VIII) sind allgemein bekannte Verbindungen der organischen Chemie.

Die zur Durchführung des erfindungsgemäßen Verfahrens (b) als Ausgangsstoffe benötigten 1-Arylpyrazole sind durch die Formel (Ia) allgemein definiert. In dieser Formel (Ia) stehen $R^1$, $R^2$, $R^3$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-pyrazole der Formel (Ia) sind erfindungsgemäße Verbindungen und erhältlich mit Hilfe des erfindungsgemäßen Verfahrens (a).

Als Verdünnungsmittel zur Durchführung des erfindungsgemäßen Verfahrens (a) kommen inerte organische Lösungs mittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole wie Methanol, Ethanol oder Propanol.

Das erfindungsgemäße Verfahren (a) wird gegebenenfalls in Gegenwart eines geeigneten Katalysators durchgeführt. Als solche kommen insbesondere anorganische Mineralsäuren oder organische Säuren wie beispielsweise Salzsäure, Schwefelsäure oder p-Toluolsulfonsäure infrage.

Es ist auch möglich, die als Ausgangsstoffe infrage kommenden Arylhydrazine der Formel (II) in Form von entsprechenden Säureadditionssalzen, wie beispielsweise Hydrochloriden einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 180 °C, vorzugsweise bei Temperaturen zwischen 20 °C und 120 °C.

Zur Durchführung des erfindungsgemäßen Verfahrens (a) setzt man pro Mol an Arylhydrazin der Formel (II) bzw. an einem entsprechenden Säureadditionssalz im allgemeinen 0.5 bis 10.0 Mol an 1,3-Diketon der Formel (III) und gegebenenfalls 0.01 bis 1.0 Mol an saurem Katalysator ein. Die Reaktionsdurchführung, Aufarbeitung und Isolierung der 1-Arylpyrazole der Formel (Ia) erfolgt nach allgemein üblichen Methoden.

Bei Verwendung von 1,3-Diketonen der Formel (III), bei welchen der Substituent $R^1$ verschieden von dem Substituenten $R^3$ ist, erhält man in der Regel Isomerengemische aus Verbindungen der Formel (Ia),

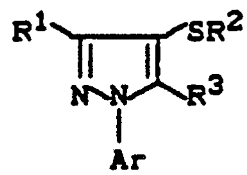

(Ia)

und Verbindungen der Formel (IX),

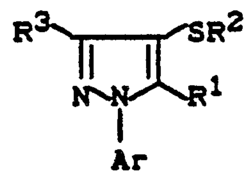

(IX)

wobei
$R^1$, $R^2$, $R^3$ und Ar in beiden Formeln die oben angegebene Bedeutung haben.

Aus diesen Isomerengemischen lassen sich mit üblichen Trennverfahren (Destillation, Kristallisation, Chromatographie) die gewünschten Reaktionsprodukte der Formel (Ia) isolieren.

Als Oxidationsmittel zur Durchführung des erfindungsgemäßen Verfahrens (b) kommen alle üblichen zur

Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (b) kommen ebenfalls inerte organische Lösungsmittel infrage.
Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (b) kann gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -actate oder -carbonate, wie beispielsweise Calciumhydroxid, Natriumhydroxid, Natriumacetat oder Natriumcarbonat.

Das erfindungsgemäße Verfahren (b) kann gegebenenfalls in Gegenwart eines geeigneten Katalysators durchgeführt werden. Als solche kommen alle üblicherweise für derar tige Schwefeloxidationen gebräuchlichen Metallsalz-Katalysatoren in Frage. Beispielhaft genannt sei in diesem Zusammenhang Ammoniummolybdat.

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (b) setzt man pro Mol an 1-Aryl-pyrazol der Formel (Ia) im allgemeinen 0.8 bis 1.2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an 1-Arylpyrazol der Formel (Ia) im allgemeinen 1.8 bis 3.0 Mol, vorzugsweise doppelt molare Mengen an Oxidationsmittel ein. Die Reaktionsführung, Aufarbeitung und Isolierung der Endprodukte der Formel (Ib) erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle odr einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum Hyalopterus arundinis, Macrosiphum avenae, Myzus spp., Phorodon humuli, Rhopa losiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea,

Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Endo- und Ektoparasiten wirksam.

Die erfindungsgemäßen Wirkstoffe zeichnen sich durch eine hohe insektizide Wirksamkeit aus. Sie lassen sich mit besonders gutem Erfolg gegen pflanzenschädigende Insekten, wie beispielsweise gegen die Larven der Meerrettichblattkäfer (Phaedon cochleariae) einsetzen. Daneben eignen sie sich auch hervorragend zur Bekämpfung von Bodeninsekten.

Außerdem besitzen die erfindungsgemäßen Wirkstoffe eine hohe Wirkung gegen Hygiene- und Vorratsschädlinge und lassen sich beispielsweise zur Bekämpfung der Stubenfliege (Musca domestica) einsetzen. Darüberhinaus lassen sich die erfindungsgemäßen Wirkstoffe mit besonders gutem Erfolg zur Bekämpfung von parasitisch lebenden Warmblüterschädlingen wie beispielsweise gegen die Weideviehfliege (Musca autumnalis), gegen Stechfliegen (Stomoxys calcitrans) oder gegen den endoparasitisch lebenden Nematoden der Gattung Caenorhabditis elegans einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorgansichen und organischen Mehlen sowie Granulate aus organischem Material wie

Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch dei Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der folgenden Beispiele erläutert werden.

Herstellungsbeispiele

Beispiel 1

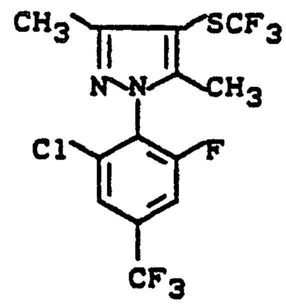

(Verfahren a)

9,2 g (0,04 Mol) 2-Chlor-6-fluor-4-trifluormethylphenylhydrazin und 8 g (0,04 Mol) 3-Trifluormethylthio-pentan-2,4-dion (vgl. Chem. Ber. 106, 1418-1422 [1973]) in 100 ml Ethanol werden 24 Stunden unter Rückfluß erhitzt. Danach setzt man 4 ml konzentrierte Schwefelsäure zu und erhitzt für weitere 6 Stunden auf 60 °C. Zur Aufarbeitung engt man die Reaktionsmischung im Vakuum ein, nimmt den Rückstand in Dichlormethan auf, wäscht mit wässriger Natriumbicarbonatlösung, trocknet über Natriumsulfat und entfernt das Lösungsmittel im Vakuum.

Man erhält 12 g (80 % der Theorie) an 1-(2-Chlor-6-fluor-4-trifluormethylphenyl)-3,5-dimethyl-4-trifluormethylthiopyrazol vom Schmelzpunkt 44 °C - 45 °C.

Beispiel 2

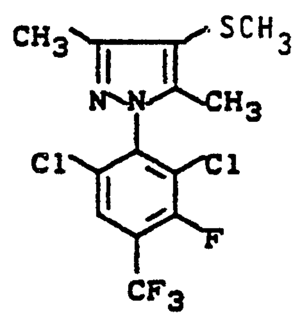

(Verfahren a)

4,38 g (0,03 Mol) 3-Methylthio-pentan-2,4-dion, 7,9 g (0,03 Mol) 2,6-Dichlor-3-fluor-4-trifluormethylphenylhydrazin und 0,1 g p-Toluolsulfonsäure werden in 200 ml Toluol 6 Stunden über einem Wasserabscheider unter Rückfluß erhitzt. Die erkaltete Reaktionsmischung wird zweimal mit Wasser gewaschen, über Magnesiumsulfat getrocknet und im Vakuum eingeengt. Der Rückstand kristallisiert beim Verrühren mit Petrolether.

Man erhält 11 g (98 % der Theorie) an 1-(2,6-Dichlor-3-fluor-4-trifluormethyl-phenyl)-3,5-dimethyl-4-methylthio-pyrazol vom Schmelzpunkt 66 °C - 69 °C.

Herstellung der Ausgangsverbindung

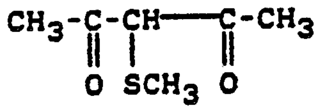

(III-1)

Zu 205 g (1,14 Mol) einer 30prozentigen methanolischen Natriummethylatlösung gibt man unter Kühlung 114 g (1,14 Mol) 2,4-Pentandion und anschließend tropfenweise unter Rühren bei 10 °C 143,5 g (1,14 Mol) Methansulfonsäuremethylthioester, rührt nach beendeter Zugabe weitere 2 Stunden bei Raumtemperatur, gießt dann die Reaktionsmischung in 2,5 l Wasser, säuert mit verdünnter Salzsäure an und extrahiert mehrfach mit Dichlormethan. Die vereinigten organischen Phasen werden über Magnesiumsulfat getrocknet, im Vakuum eingeengt und im Hochvakuum destilliert.

Man erhält 98,2 g (59 % der Theorie) an 3-Methylthio-2,5-pentandion vom Siedepunkt 46 °C bei 0,8 mbar.

Beispiel 3

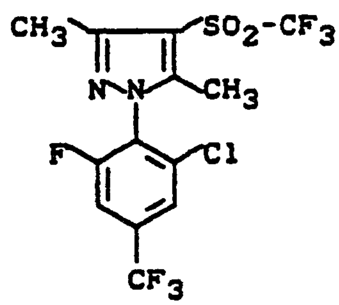

(Verfahren b)

Zu 3,8 g (0,01 Mol) 1-(2-Chlor-6-fluor-4-trifluormethyl-phenyl)-3,5-dimethyl-4-trifluormethylthio-pyrazol in 50 ml Dichlormethan gibt man portionsweise 4,3 g (0,02 Mol) m-Chlorperbenzoesäure, rührt anschließend 20 Stunden bei 40 °C, verdünnt mit Dichlormethan, wäscht mit gesättigter wässriger Natriumhydrogencarbonatlösung, trocknet über Magnesiumsulfat und engt im Vakuum ein. Der Rückstand wird chromatographisch gereinigt (Kieselgel; Laufmittel: Dichlormethan/Hexan 7 : 3).

Man erhält 3,1 g (90 % der Theorie) an 1-(2-Chlor-6-fluor-4-trifluormethyl-phenyl)-3,5-dimethyl-4-trifluormethylsulfonyl-pyrazol vom Schmelzpunkt 88 °C.

In entsprechender Weise und gemäß den allgemeinen Angaben zur Herstellung erhält man die folgenden 1-Arylpyrazole der allgemeinen Formel (I):

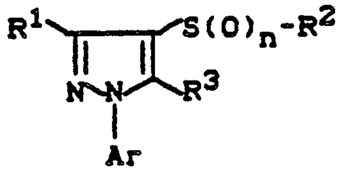 (I)

| Bsp. Nr. | $R^1$ | $-S(O)_n-R^2$ | $R^3$ | Ar | physikalische Eigenschaften |
|---|---|---|---|---|---|
| 4 | $CH_3$ | $-SCCl_2F$ | $CH_3$ | 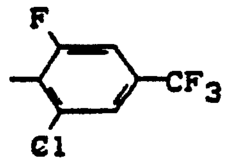 | Fp 59-60° C |
| 5 | $CH_3$ | $-SCF_3$ | $CH_3$ | 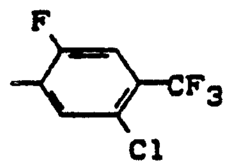 | $n_D^{20}$ 1,4998 |
| 6 | $CH_3$ | $-SO_2-CCl_2F$ | $CH_3$ | 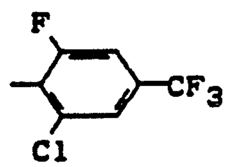 | Fp 124-125° C |
| 7 | $CH_3$ | 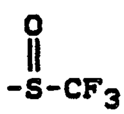 | $CH_3$ | 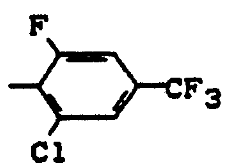 | $n_D^{20}$ 1.4943 |

| Bsp. Nr. | $R^1$ | $-S(O)_n-R^2$ | $R^3$ | Ar | physikalische Eigenschaften |
|---|---|---|---|---|---|
| 8 | $CH_3$ | $-SCH_3$ | $CH_3$ | 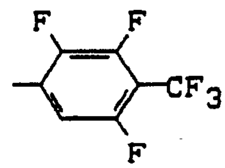 | Fp 33-34° C |
| 9 | $CH_3$ | $-SC_2H_5$ | $CH_3$ | 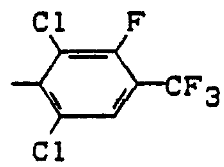 | $n_D^{20}$ 1,5348 |
| 10 | $CH_3$ | $-SCH_3$ | $CH_3$ | 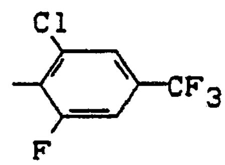 | Fp 56-57° C |
| 11 | $CH_3$ | $-SCF_3$ | $CH_3$ | 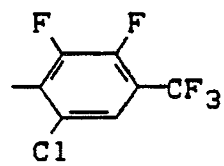 | Fp 55-60° C |
| 12 | $CH_3$ | $-SCClF_2$ | $CH_3$ | 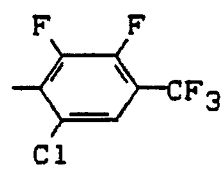 | $n_D^{20}$ 1,5130 |

Fp = Schmelzpunkt

$n_D^{20}$ = Brechungsindex

Herstellung der Ausgangsstoffe

Beispiel II-1

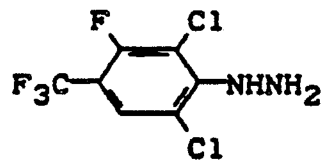

In 1000 ml Ethanol werden 470 g (1,87 Mol) 3,5-Dichlor-2,4-difluorbenzotrifluorid vorgelegt und 142 g (2,84 Mol) Hydrazinhydrat zudosiert und anschließend für 3 Stunden unter Rückfluß erhitzt. Danach wird das Lösungsmittel unter reduziertem Druck abdestilliert und der Rückstand in 1 l kaltes Wasser eingerührt. Nach 30 Minuten wird abgesaugt und das Festprodukt im Umluftschrank getrocknet.

Man erhält 445 g (90 % der Theorie) 2,6-Dichlor-3-fluor-4-trifluormethyl-phenylhydrazin mit einem Schmelzpunkt von 50 °C bis 51 °C.

Beispiel II-2

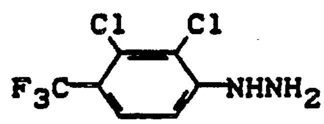

Es werden 100 g (0,4 Mol) 2,3,4-Trichlorbenzotrifluorid vorgelegt, 200 ml Pyridin und anschließend 100 g (2,0 Mol) Hydrazinhydrat zugefügt und dann 12 Stunden auf Rückflußtemperatur erhitzt. Danach wird das Pyridin zu 90 % abdestilliert und der verbleibende Rückstand in 250 ml Wasser eingerührt. Das kristalline Produkt wird abgesaugt, mit etwas Wasser gewaschen und getrocknet.

Man erhält 78 g (80 % der Theorie) 2,3-Dichlor-4-trifluormethyl-phenylhydrazin mit einem Schmelzpunkt von 79 °C bis 80 °C.

In entsprechender Weise und gemäß den allgemeinen Angaben zur Herstellung erhält man die folgenden Arylhydrazine der Formel (II),

$$Ar-NH-NH_2 \qquad (II)$$

| Bsp. Nr. | Ar | Schmelzpunkt /°C |
|---|---|---|
| II-3 | 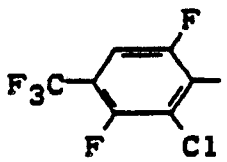 | 72-73 |
| II-4 | 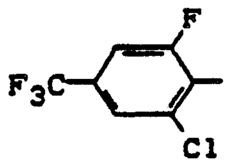 | 60-61 |
| II-5 | 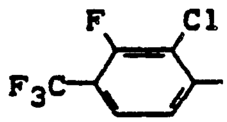 | 102-103 |
| II-6 | 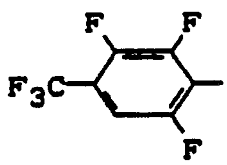 | 60-62 |
| II-7 | 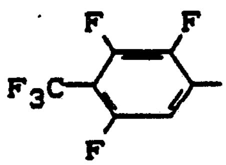 | 69-70 |
| II-8 | 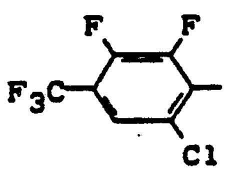 | 80 |

| Bsp. Nr. | Ar | Schmelzpunkt /°C |
|---|---|---|
| II-9 | 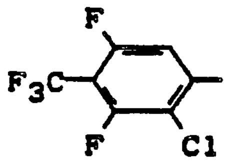 | 82-84 |
| II-10 | 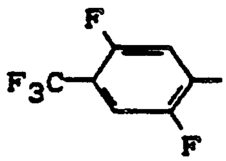 | 41 |
| II-11 | 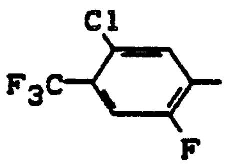 | 69-70 |

Beispiel IV-1/IV-2

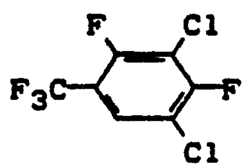

+

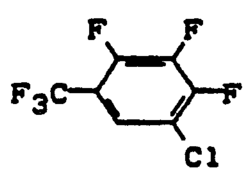

Zu 800 g (13.8 Mol) Kaliumfluorid in 1800 ml trockenem Tetramethylensulfon gibt man 900 g (3.17 Mol) 2,3,4,5-Tetrachlorbenzotrifluorid (vgl. z.B. EP 150 587) und erhitzt 10 Stunden auf 200 °C. Zur Aufarbeitung wird im Vakuum eingeengt und destilliert.

Man erhält zunächst 50 g (6,8 % der Theorie) an 2,3,4-Trifluor-5-chlor-benzotrifluorid vom Siedpunkt Kp 32 °C - 38 °C bei 10 mbar und vom Brechungsindex $n_D^{20}$ 1.4130 und als 2. Fraktion 490 g (61.6 % der Theorie) an 2,4-Difluor-3,5-dichlorbenzotrifluorid vom Siedepunkt 57 °C - 59 °C bei 10 mbar und vom Brechungsindex $n_D^{20}$ 1,4510.

Beispiel IV-3

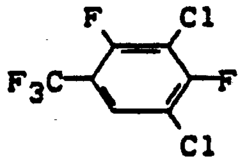

252,5 g (1 Mol) 3-Chlor-2,4,5,6-tetrafluorbenzotrifluorid (vgl. z.B. Zh. obshch. Khim. 37, 1686-1687 [1967]) und 86 g (1.05 Mol) Natriumacetat in 1000 ml Eisessig werden in Gegenwart von 10 g Palladium auf Aktivkohle (5 %) bei einem Wasserstoffdruck von 30 bis 50 bar und 120 °C bis zur Druckkonstanz hydriert. Zur Aufarbeitung filtriert man aus der erkalteten Reaktionsmischung den Katalysator ab und destilliert den Rückstand.

Man erhält 210 g (96 % der Theorie) an 2,3,4,6-Tetrafluorbenzotrifluorid vom Siedepunkt 105 °C - 106 °C und vom Brechungsindex $n_D^{20}$ 1.3770.

In entsprechender Weise erhält man die folgenden Arylhalogenide der Formel (IV),

$$Ar-Hal^1 \qquad (IV)$$

| Bsp. Nr. | Ar | $Hal^1$ | physikalische Konstanten |
|---|---|---|---|
| IV-4 | 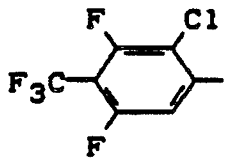 | F | Kp 145° C/760<br>$n_D^{20}$=1.4170 |
| IV-5 | 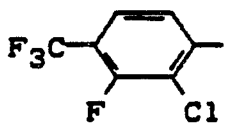 | F | Kp 37° C/16<br>$n_D^{20}$=1.4268 |
| IV-6 | 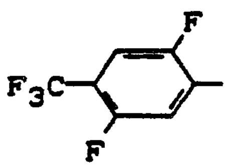 | F | Kp 104° C/760<br>$n_D^{20}$=1.3862 |
| IV-7 | 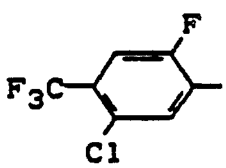 | F | Kp 130-140° C/760<br>$n_D^{20}$=1.4250 |

<u>Erklärung:</u>

Kp = Siedepunkt / mbar

$n_D^{20}$ = Brechungsindex

Anwendungsbeispiele

In den folgenden Anwendungsbeispielen wurden die nachstehend aufgeführten Verbindungen als Vergleichssubstanzen eingesetzt:

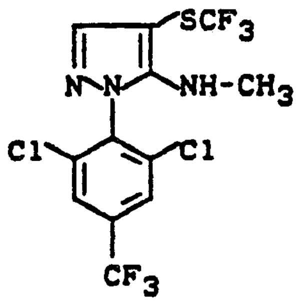

(A)

5-Methylamino-4-trifluormethylthio-1-(2,6-dichlor-4-trifluormethyl-phenyl)-pyrazol

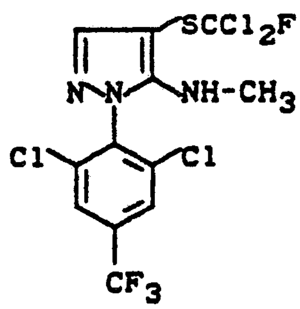

(B)

5-Methylamino-4-dichlorfluormethylthio-1-(2,6-dichlor-4-trifluormethyl-phenyl)-pyrazol

(beide bekannt aus EP 201 852)

Beispiel A

$LT_{100}$-Test für Dipteren

Testtiere: Musca domestica, resistent

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt solange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1 und 4.

## Beispiel B

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigt z.B. die folgende Verbindung der Herstellungsbeispiele überlegene Wirksamkeit gegenüber dem Stand der Technik: 4.

## Beispiel C

Test mit Stomoxys calcitrans

Emulgator: 35 Gewichtsteile Ethylenglykolmonomethylether

35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigt z.B. die folgende Verbindung der Herstellungsbeispiele überlegene Wirkung gegenüber dem Stand der Technik: 4.

Beispiel D

Facefly - Test (Musca autumnalis)

Lösungsmittel: 35 Gewichtsteile Ethylenglykolmonomethylether

35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad in Prozent bestimmt, wobei 100% bedeuten, daß alle und 0%, daß keine Fliegen abgetötet worden sind.

Bei diesem Test zeigt z.B. die folgende Verbindung der Herstellungsbeispiele überlegene Wirkung gegenüber dem Stand der Technik: 4.

Beispiel E

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wird auf eine Replica-Platte gegeben. Dazu gibt man 2 ml einer E.coli-Suspension, zu der man 10 - 20 weibliche Tiere oder Larven von Caenorhabditis elegans in 0,5 ml steriler M9 Pufferlösung gegeben hat. Die E.coli-Suspension wird hergestellt, indem man 300 ml einer Übernachtkultur eines Uracil-bedürftigen E.coli-Stammes mit 1,8 l steriler M9 Pufferlösung versetzt.

Der Versuchsansatz wird 7 Tage bei 22 °C inkubiert und danach ausgewertet. Es wird bewertet, inwieweit der Wirkstoff die Vermehrung beeinträchtigt und die Konzentration angegeben bei der die Vemehrung verhindert wird.

Bei diesem Test zeigen z.B. die folgenden Verbindungen der Herstellungsbeispiele eine mindestens 95 %ige Hemmung der Vermehrung des Nematoden C. elegans
- bei einer Konzentration von $\leq$ 100 µg/ml: 1 und 4.

Beispiel F

Grenzkonzentrations-Test / Bodeninsekten

Testinsekt: Phorbia antiqua-Maden im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele eine mindestens 95 %ige Abtötung der Bodenmaden bei einer Wirkstoffkonzentration von 2,5 ppm: 1, 3, 4, 6, 7.

## Tabelle F

Bodeninsektizide

Phorbia antiqua-Maden im Boden

| Wirkstoff (Konstitution) | Abtötungsgrad in % bei Wirkstoffkonzentration in ppm |
|---|---|
| 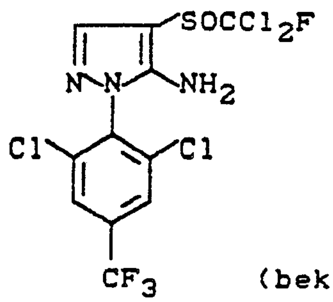 (bekannt) | 2,5 ppm = 0 % |
| 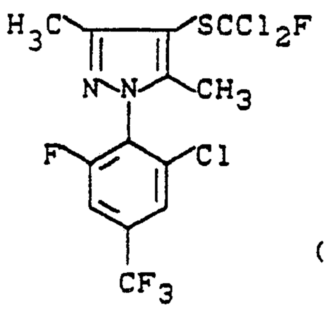 (4) | 2,5 ppm = 95 % |
| 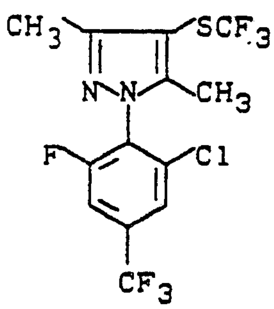 (1) | 2,5 ppm = 95 % |

T a b e l l e F (Fortsetzung)

Bodeninsektizide

Phorbia antiqua-Maden im Boden

| Wirkstoff (Konstitution) | Abtötungsgrad in % bei Wirkstoffkonzentration in ppm |
|---|---|
| 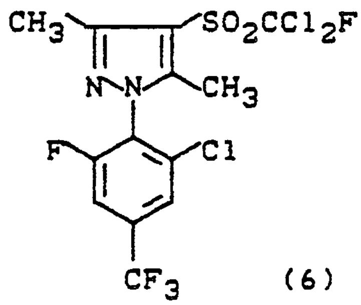 (6) | 2,5 ppm = 95 % |
| 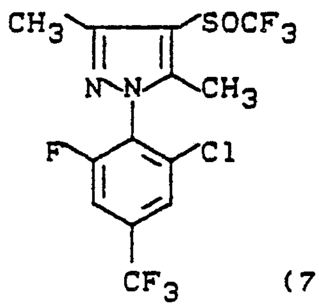 (7) | 2,5 ppm = 95 % |
| 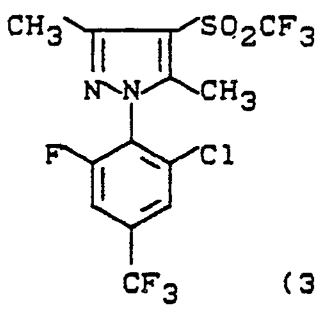 (3) | 2,5 ppm = 95 % |

Beispiel G

Grenzkonzentrations-Test / Bodeninsekten

Testinsekt: Diabrotica balteata - Larven im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche im ppm (= mg/l) angegeben wird. Man füllt den Boden in 0,5 l Töpfe und läßt diese bei 20°C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie in der unbehandelten Kontrolle.

Bei diesem Test zeigen z.B. die folgenden Verbindungen der Herstellungsbeispiele eine mindestens 95 %ige Abtötung der Bodenlarven bei einer Wirkstoffkonzentration von 5 ppm: 1, 7.

<u>Tabelle G</u>

Bodeninsektizide

<u>Diabrotica balteata - Larven im Boden</u>

| Wirkstoff (Konstitution) | Abtötungsgrad in % bei Wirkstoffkonzentration in ppm |
|---|---|
| 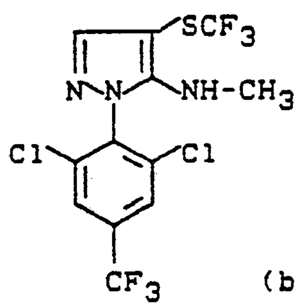 (bekannt) | 5 ppm = 0 % |
| 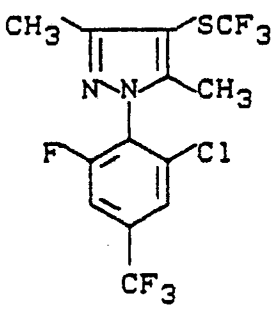 (1) | 5 ppm = 95 % |
| 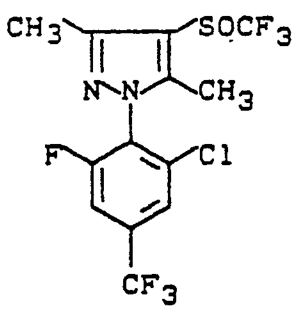 (7) | 5 ppm = 95 % |

Beispiel H

Grenzkonzentrations-Test / Wurzelsystemische Wirkung

Testinsekt: Phaedon cochleariae-Larven

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele eine 100 %ige Abtötung der Phaedon cochleariae-Larven bei einer Wirkstoffkonzentration von 10 ppm: 7, 10.

Tabelle H

Wurzelsystemische Wirkung

Phaedon cochleariae-Larven

| Wirkstoff (Konstitution) | Abtötungsgrad in % bei Wirkstoffkonzentration in ppm |
| --- | --- |
| 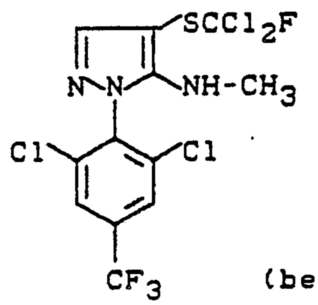 (bekannt) | 10 ppm = 0 % |
| 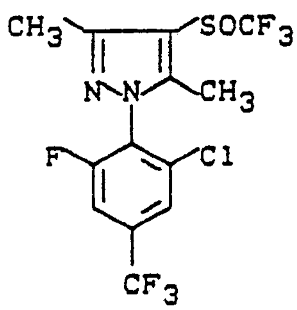 (7) | 10 ppm = 100 % |
| 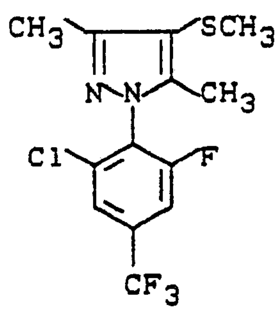 (10) | 10 ppm = 100 % |

**Ansprüche**

1) 3,5-Dialkyl-1-arylpyrazole der allgemeinen Formel (I),

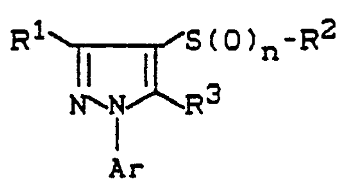

(I)

in welcher

$R^1$ für Alkyl steht,

$R^2$ für Alkyl oder Halogenalkyl steht,
$R^3$ für Alkyl steht,
Ar für einen der Reste

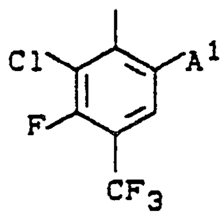 ; 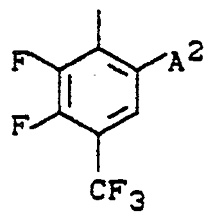 ; 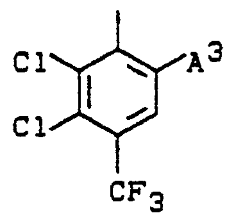 ;

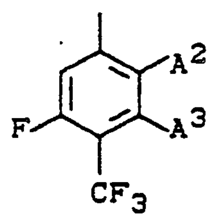 ; 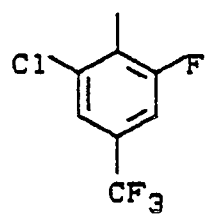 oder 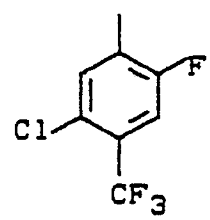

steht, wobei
$A^1$ für Wasserstoff, Fluor oder Chlor steht,
$A^2$ für Fluor oder Chlor steht,
$A^3$ für Wasserstoff oder Fluor steht und
n für eine Zahl 0, 1 oder 2 steht.

2) 3,5-Dialkyl-1-arylpyrazole der allgemeinen Formel (I) gemäß Anspruch 1), in welcher
$R^1$ für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,
$R^3$ für geradkettiges oder verzweigtes Alkyl mit 1 bis 6 Kohlenstoffatomen steht,
Ar für einen der Reste

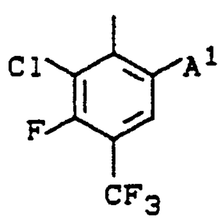 ; 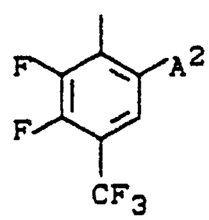 ; 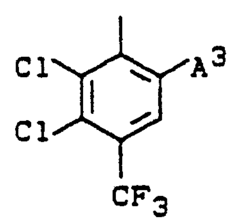 ;

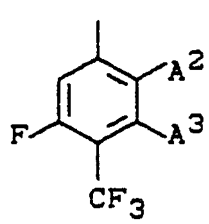 ; 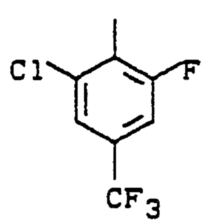 oder 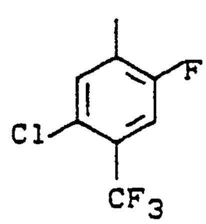

steht, wobei
$A^1$ für Wasserstoff, Fluor oder Chlor steht,
$A^2$ für Fluor oder Chlor steht,
$A^3$ für Wasserstoff oder Fluor steht und
n für eine Zahl 0, 1 oder 2 steht.

3. 3,5-Dialkyl-1-arylpyrazole der allgemeinen Formel (I) gemäß Anspruch 1), in welcher

$R^1$ für Methyl oder Ethyl steht,

$R^2$ für Methyl, Ethyl n- oder i-Propyl, n-, i-, s-oder t-Butyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl oder Brompropyl steht,

$R^3$ für geradkettiges oder verzweigtes Alkyl mit 1 bis 4 Kohlenstoffatomen steht,

Ar für einen der Reste

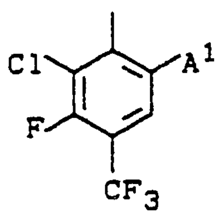 ; 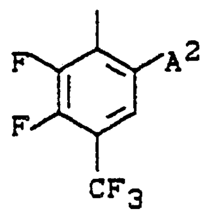 ; 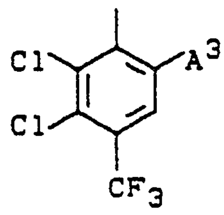 ;

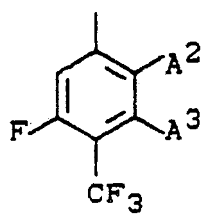 ; 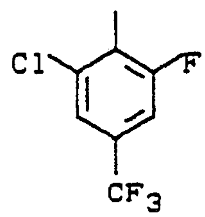 oder 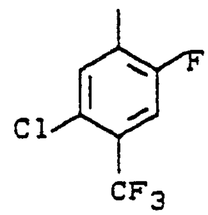 .

steht, wobei

$A^1$ für Wasserstoff, Fluor oder Chlor steht,

$A^2$ für Fluor oder Chlor steht,

$A^3$ für Wasserstoff oder Fluor steht und

n für eine Zahl 0, 1 oder 2 steht.

4. 3,5-Dialkyl-1-arylpyrazole der allgemeinen Formel I gemäß Anspruch 1) in welcher

$R^1$ für Methyl steht,

$R^2$ für Methyl, Ethyl, Trifluormethyl, Dichlorfluormethyl oder Difluorchlormethyl steht,

$R^3$ für Methyl, Ethyl, n- oder i-Propyl steht,

Ar für einen der Reste

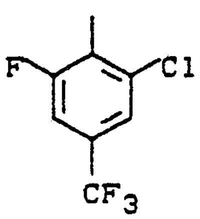 ; 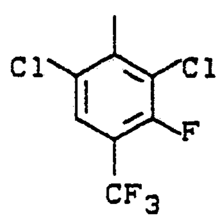 oder 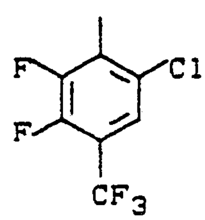 steht

und

n für eine Zahl 0, 1 oder 2 steht.

5) Verfahren zur Herstellung von 3,5-Dialkyl-1-arylpyrazolen der allgemeinen Formel (I)

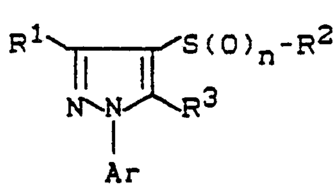 (I)

in welcher
$R^1$ für Alkyl steht,
$R^2$ für Alkyl oder Halogenalkyl steht,
$R^3$ für Alkyl steht,
Ar für einen der Reste

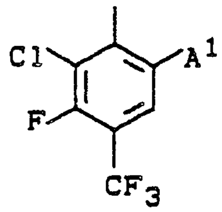 ; 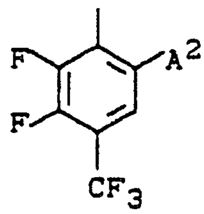 ; 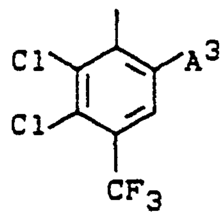 ;

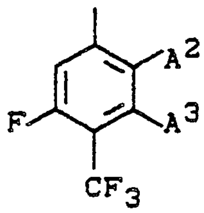 ; 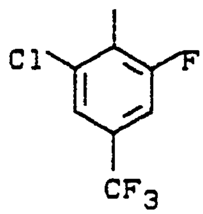 oder 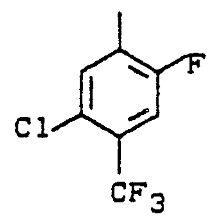

steht, wobei
$A^1$ für Wasserstoff, Fluor oder Chlor steht,
$A^2$ für Fluor oder Chlor steht,
$A^3$ für Wasserstoff oder Fluor steht und
n für eine Zahl 0, 1 oder 2 steht,
dadurch gekennzeichnet, daß man

a) zum Erhalt von 3,5 Dialkyl-1-arylpyrazolen der allgemeinen Formel (Ia),

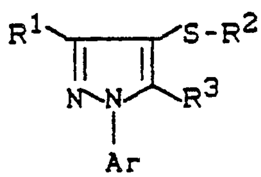

(Ia)

in welcher
$R^1$, $R^2$, $R^3$ und Ar die oben angegebene Bedeutung haben,
Arylhydrazine der Formel (II),
$Ar\text{-}NH\text{-}NH_2$ (II)
in welcher
Ar die oben angegebene Bedeutung hat,
oder deren Säureadditionssalze mit 1,3-Diketonen der Formel (III),

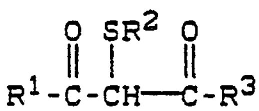

(III)

in welcher
$R^1$, $R^2$ und $R^3$ die oben angegebene Bedeutung haben,
gegebenenfalls in Gegenwart eines Verdünnungsmittel und gegebenenfalls in Gegenwart eines Katalysators umsetzt,
oder daß man

b) zum Erhalt von 4-Sulfinyl- und 4-Sulfonyl-3,5-deialkyl-1-aryl-pyrazole der Formel (Ib)

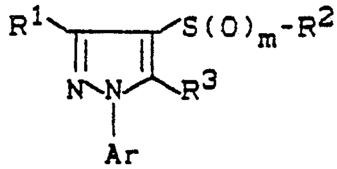

(Ib)

in welcher
$R^1$, $R^2$, $R^3$ und Ar die oben angegebene Bedeutung haben und
m für eine Zahl 1 oder 2 steht,
die nach Verfahren (a) erhältlichen 3,5-Dialkyl-1-arylpyrazole der Formel (Ia),

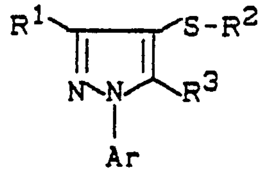

(Ia)

in welcher
$R^1$, $R^2$, $R^3$ und Ar die oben angegebene Bedeutung haben,
mit Oxidationsmitteln gegebenenfalls in Gegenwart eines Verdünnungsmittels und gegebenenfalls in Gegenwart eines Katalysators oxidiert.

6) Schädingsbekämpfungsmittel, gekennzeichnet durch einen Gehalt an mindestens einem 3,5-Dialkyl-1-arylpyrazol der Formel (I).

7) Insektizide Mittel, gekennzeichnet durch einen Gehalt an mindestens einem 3,5-Dialkyl-1-arylpyrazol der Formel (I).

8) Verfahren zur Bekämpfung von tierischen Schädlingen, insbesondere von Insekten, dadurch gekennzeichnet , daß man 3,5-Dialkyl-1-arylpyrazole der Formel (I) auf tierische Schädlinge und/oder deren Lebensraum einwirken läßt.

9) Verwendung von 3,5-Dialkyl-1-arylpyrazolen der Formel (I) zur Bekämpfung von tierischen Schädlingen, insbesondere von Insekten.

10) Verfahren zur Herstellung von Mitteln gegen tierische Schädlinge, dadurch gekennzeichnet, daß man 3,5-Dialkyl-1-arylpyrazole der Formel (I) mit Streckmitteln und/oder oberflächenaktiven Mitteln vermischt.

European Patent Office

# EUROPEAN SEARCH REPORT

Application Number

EP 88 11 1845

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | DE-A-3 600 287 (BAYER AG) * Ansprüche; Beispiele 51,52 in Zusammenhang mit Beispiel 1,46 * | 1-10 | C 07 D 231/18<br>A 01 N 43/56 //<br>C 07 C 149/14<br>C 07 C 109/04<br>C 07 C 25/13 |
| A | EP-A-0 216 102 (BAYER AG) * Ansprüche 1-3,5-9; Beispiele 14,18,23,24,26,46,71-73,102 * | 1,6-10 | |
| A | WO-A-8 703 781 (MAY & BAKER LTD.) * Ansprüche 1-3,5-8,10,12 * | 1,5-10 | |
| A,P<br>A,D | EP-A-0 201 852 (BAYER AG) * Ansprüche 1,3-5 * | 1,6-10<br>1,6-10 | |
| A | EP-A-0 224 831 (BAYER AG) * & DE - A - 3 617 977 (Kat. D) * | | |
| | | | TECHNICAL FIELDS SEARCHED (Int. Cl.3) |
| | | | C 07 D 231/00<br>A 01 N 43/00 |

The present search report has been drawn up for all claims

| Place of search | Date of completion of the search | Examiner |
|---|---|---|
| BERLIN | 27-10-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)